# EUROPEAN PATENT APPLICATION

(11) **EP 4 524 989 A1**
(43) Date of publication of application: **19.03.2025**
(21) Application number: 23196942.9
(22) Date of filing: 12.09.2023
(51) Int. Cl.: G16H 40/63, G06F 3/0488, A61M 5/14, G16H 20/17

(54) **SIMPLIFIED USER INTERFACE FOR MEDICAL INFUSION DEVICE**

(71) Applicant: Ypsomed AG, 3401 Burgdorf (CH)
(72) Inventor: Weinhold, Thomas, 3007 Bern (CH); Gertsch, Lukas, 4500 Solothurn (CH)
(74) Representative: Meier Obertüfer, Jürg

(57) **Abstract**

The present invention is concerned with a portable medical infusion pump comprising a user interface with at least a touch sensitive display and a button. The portable medical infusion pump further comprises an electronic controlling assembly for operating and controlling the portable medical infusion pump. The electronic controlling assembly of the portable medical infusion pump is configured to put the user interface from locked state in an unlocked state through an unlock process performed by a using person. The unlock process preventing inadvertent operation of the portable medical infusion pump.

## Description

### FIELD OF THE INVENTION

The present invention relates to the user interface of a portable medical infusion pump.

### BACKGROUND OF THE INVENTION

Portable ambulatory medical devices have proved useful for treating patients with medical conditions that require continuous monitoring and/or treatment. One example of such a portable ambulatory medical device is a device that involves the delivery of fluids. There are many applications in academic, industrial, and medical fields, as well as others, that involve devices capable of accurately and controllably delivering fluids, including liquids and gases, that have a beneficial effect when administered in known and controlled quantities. This is particularly true in the medical field, where treatments for many patients include the administration of a known amount of a substance at predetermined intervals. For example, the treatment of diabetes involves just such a regimented dosage of medicaments such as insulin. In addition, diabetes is one medical indication wherein patients routinely administer the medicament to themselves by a subcutaneous modality, such as a hypodermic syringe injection or by an ambulatory infusion pump. As such, providing a patient with the means to safely, reliably, and comfortably administer required doses of medication such as, e.g., insulin, may be particularly important in order to facilitate patient compliance and accurate treatment of the condition.

Some ambulatory medical devices include a touchscreen on which symbols may be displayed and from which inputs may be received for operation of the device. Other input mechanisms involve keyboards or hardware switches. In general, a series of display screens or windows are shown on a device display or on the device touchscreen, showing alphanumeric text and symbols, thereby providing menu screens through which the user can control operation of the device. User interaction, such as by touching the alphanumeric text and symbols, provides user input and facilitates navigation through the menu screens and selection and controlling the device functions.

The phenomenon of unintended, inadvertent activation of portable devices is not an uncommon occurrence. Telephone calls accidentally placed via a mobile telephone through inadvertent activation have become a fact of modern life. Such accidental calls can be annoying and troublesome for a mobile telephone user. In the case of a portable ambulatory medical device, such accidental activation can have serious consequences. In fact, in the case of portable ambulatory medical devices, any changes at all that are unintended or inadvertent may be problematic and even dangerous. For example, an untimely delivery of insulin, or delivery of an unexpectedly changed amount of insulin, or the absence of an expected dose, can have extremely deleterious results, and may even be dangerous to the user. User safety (and also usability) would be improved with a reduction in the likelihood of an accidental or unintended activation or deactivation of a portable ambulatory medical device.

US2020012401 A1 discloses an infusion pump with a touch screen. In order to prevent inadvertent operation of the pump the touch screen has a locked and an unlocked state. The pump comprises a processor which is configured to put the touch screen into the locked state which limits input received into the touch screen. It is further configured to display an unlock screen on the touch screen and subsequently to unlock the touch screen from the locked state to the unlocked state upon receiving a predetermined activation sequence comprising a sequence of predetermined alphanumeric characters selected through the touch screen. Activation sequence means that the displayed alphanumeric characters have to be touched in the correct sequence in order to make the processor changing the state. Illiterate people and younger children may be confused by the displayed alphanumerical characters on the unlock screen and may therefore not be able to unlock the touch screen.

Hence there is a need for simple user interfaces of infusion pumps, in particular in providing simplified but safe methods to firstly prevent inadvertent operation of the infusion pump and secondly, to unlock locked interfaces.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

The term "medical infusion pump" preferably refers to portable medical infusion pumps.

The term "using person" refers to a potential patient wearing and using device, but it refers to person assisting a patient (like a health care professional) and hence just operating a device.

### DESCRIPTION OF THE INVENTION

It is an objective of the invention to provide a portable medical infusion pump with a user interface providing simplified user interaction. It is a further object of the invention to provide a method for unlocking the user interface of a portable medical infusion pump by a few simple steps while maintaining protection from inadvertent use.

The objectives are achieved by the medical infusion pump and the method to unlock a portable medical infusion pump as disclosed in the independent claims. Further embodiments of the invention are disclosed in the dependent claims, the description and the drawings.

In one aspect of the invention, the invention comprises a portable medical infusion pump configured to deliver fluid medicament to an using person. The medical infusion pump may be an ambulatory infusion pump. For example, the infusion pump may be an insulin pump similar to the YpsoPump marketed by Ypsomed in many European countries. The medical infusion pump according to the invention comprises a user interface comprising at a depressible button and a touch sensitive display. The touch sensitive display may be a combination of a transparent touch sensitive layer and an LCD or OLED display capable of displaying background, characters or symbols in black and white, a single color in varying shades or in full color. Further, the portable medical infusion pump comprises an electronic controlling assembly. The electronic controlling assembly comprises a microcontroller or processor configured to execute instruction or programs stored in a part of the electronic controlling assembly, for example a non-transitory computer readable medium or memory. The electronic controlling assembly may be seen as small and lean computer. The electronic controlling assembly is functionally coupled to the user interface, including the button and the touch sensitive display. The electronic controlling assembly is configured to create and display screens on the touch sensitive display as well receive signal from the same. Moreover, the electronic controlling assembly is configured to receive signals from the button. The microcontroller or processor is configured to process received signals and in reaction create and display an according screen on the touch sensitive display.

The depressible button is to be construed in its broadest sense as a push button wherein a depression of the button is an actual geometrically detectable movement of at least a part of the button or on the other side depression may be a kind of touch or pressure detection, where no movement is visible. Important in this context is, that the button is functionally coupled to the controlling assembly, capable of signaling a button depression and a duration of the depression. One depression event is a continuous depression (as the button is a push button and not a switch).

As a using person operates the portable medical infusion pump through the user interface with the touch sensitive display, the electronic controlling assembly is further configured to prevent inadvertent operation of the medical infusion pump by putting the user interface into a locked mode (or state) when the using person is not interacting with the user interface on purpose. In the locked mode only restricted interaction through the user interface is possible. The medical infusion pump has to undergo an unlock process when the using person intentionally wants to interact with the touch sensitive display. In a first step the touch sensitive display is activated and optionally a status screen is displayed on the touch sensitive display . This is done through depression of the button for an at least predefined amount of time. The predefined amount of time may vary between less than a second up to several second. The electronic control assembly detects the depression as well as the duration of the depression. As a consequence of correct depression of the button for at least the predefined amount time the electronic control assembly activates the touch sensitive screen and optionally creates and displays the status screen e. g. comprising information about current or past medication delivery or filling level of a drug reservoir present in the medical infusion pump. In order to proceed with the unlocking process the using person has to touch the touch sensitive screen with a finger and perform a swipe movement with the finger on or across the touch screen. When receiving the according signal generated by the swiping movement at the electronic controlling assembly the electronic controlling assembly creates and displays a first screen on the touch sensitive display. Optionally, if the time span between depression of the button and the swipe movement exceeds a predetermined period, the electronic controlling assembly may abort the unlocking procedure and deactivate the touch sensitive screen again. The first screen comprises at least two graphical elements with one of the elements graphically emphasized or highlighted. The emphasis on one of the graphical elements is intended to attract the attention of the using person and guide the person to touch the emphasized graphical element with a finger. When the user touches the emphasized graphical element with a finger, the electronic controlling assembly receives the according signal and, as a consequence, creates and displays an updated screen. The updated screen shows the same number of graphical elements. However, the emphasized and subsequently touched graphical element is changed to a non-emphasized state and also changed graphically with the aim to graphically represent confirmation of correctly touching the emphasized graphical element (confirmed state). The changed graphical element may be called graphical confirmation element below. Moreover, one of the not yet emphasized graphical elements has become emphasized on the updated screen, and hence attracting the using person's attention. Upon touching the newly emphasized graphical element the electronic controlling assembly creates and displays another updated screen on which the newly emphasized graphical element is de-emphasized and changed to the confirmed state represented by a graphical confirmation element. Moreover, yet another of the not-yet touched elements becomes emphasized. This process of the touching and changing graphical elements is repeated until all graphical elements have reached the confirmed state. Once all graphical elements have been changed into graphical confirmation element, the electronic controlling assembly fully unlocks the user interface for further interaction of the using person with the medical infusion pump. Optionally, a user menu is created and displayed by the electronic controlling assembly on the touch sensitive display upon completion of the unlocking process. In a variant a status screen is created and shown upon completion of the unlock process and the mentioned user menu may be reached by another swipe movement with finger over the touch sensitive display.

If the using person does not complete the unlock process for any reason or within a predefined amount of time, optionally, a time-out function may be applied by the controlling assembly, the time function leading to the abortion of the unlock process.

In one aspect the graphical elements as described above do not contain alphanumeric characters. This has the advantage that illiterate people or small children do not get confused by numbers or letters possibly indicating something they don't understand.

In one aspect the emphasis of the emphasized graphical elements is realized by an increased luminosity or brightness of the emphasized graphical element on the touch sensitive display. Alternatively, the emphasis may also be added by changing the color for the emphasized graphical element. In an even further alternative, the emphasis may be added by animating the emphasized graphical element, e. g. by wiggling, blinking or rotating the emphasized graphical element. Combinations of the alternatives are of course also possible. Moreover, the kind and degree of desired emphasis may be selectable and adjustable in the user menu of the medical infusion pump in a further variant. In yet further alternatives or combinations of the above-mentioned alternatives, emphasis may be added by changing size or shape of the graphical element to be emphasized. The graphical elements may be arranged in variable orientations and arrangements on the touch sensitive display. E.g. horizontally, vertically, or diagonally distributed across the touch sensitive display. The order, in which the graphical elements become highlighted, may be varied automatically or set manually.

In one aspect, the graphical elements as described above (prior to change into the confirmed state) consist of simple geometrical shapes. Optionally frames surround each of the elements. Alternatively and optionally, one frame surrounds all graphical elements. The simple geometrical shape may be a square, a rectangle, a triangle or any other simple polygon. Preferably but optionally, the simple geometrical shape may be filled on the display to increase readability.

In one further possible aspect, the emphasized graphical element is changed into the graphical confirmation element as soon as the using person touches the graphical emphasized element with a finger. In this aspect the electronic controlling assembly just changes the emphasized graphical element into the confirmation element when receiving a touch signal from the touch sensitive display. Upon remove the finger from the touch sensitive display the full new screen is generated and displayed on the touch sensitive display. This aspect has the advantage that a using person is getting positive feedback earlier.

In one aspect of the invention the graphical confirmation elements comprise a single symbol describing confirmation or completion of an action in a figurative way, so it may be understood by the broadest possible range of users. The single symbol may include a check mark. Alternatively, the symbol may include a happy emoji or emoticon signaling confirmation. The emoji or emoticon may include a smiley or a thumbs-up symbol.

One aspect relates to operation of the medical infusion pump when in locked mode. When in locked mode a using person may only perform limited variety of operations through the user interface. In one alternative, a using person may be able to program a so-called blind bolus. If the medical infusion pump is a conventional insulin pump with an infusion set this means that a using person does not have to interact with the display to program a bolus. For example, the pump may be kept in a trouser pocket while programming the bolus. In the present aspect a using person may program a blind bolus while the user interface being in locked mode by repeatedly depressing the button. As described above, the button is also used to start the unlock procedure. In order to discriminate the different commands of starting the unlock procedure and programming a blind bolus, the blind bolus programming may be started by depressing the button using a double- or triple-click-like depressing sequence. The blind bolus programming procedure is started if the electronic controlling assembly receives an according signal of such a sequence. In order to program the actual blind bolus, the using person presses the button again for a number of times corresponding to a desired size of the blind bolus. Optionally, the electronic controlling assembly listens for button depressing signals for a predefined time span, e. g. for 20 seconds. As mentioned, the number of depression events corresponds to a size of the bolus, in some embodiments the size of the bolus scales linearly with the number of depression events. In case the medicament to be administered is insulin, one depression event may correspond to one I. U. (International Units) of insulin. Alternatively, the amount per depression may correspond to less or more than one I. U. In other alternatives with e. g. other drugs each number of depressions may correspond to a predefined amount of drug. For example, two depressions may correspond to 10 mg of drug and three depressions to 20 mg of drug. After the desired number of depressions is executed by the using person, the electronic controlling assembly operates the infusion pump to administer the programed bolus.

One aspect of the invention relates to a method to unlock the user interface of a portable medical infusion pump from a locked state, where only restricted interaction with a user is possible to an unlocked state where a user may fully operate and control the medical infusion pump. The medical infusion pump in this aspect corresponds widely to the medical infusion pump above. The (graphical) elements of the medical infusion pump in this aspect correspond to the element described above. The electronic controlling assembly of the medical infusion pump is configured to perform the steps in interaction with a using person, to unlock the user interface as follows. The electronic controlling assembly starts the unlock procedure after the button is depressed for at least a predefined amount of time. This activates the touch sensitive display of the user interface. Then, after a swipe movement of a finger of the user is detected on the touch sensitive display, the electronic controlling assembly creates and displays a first screen on the touch sensitive display, the first screen displaying at least two graphical elements, wherein one of the graphical elements is graphically emphasized relative to the other graphical elements. Subsequently, after a touch of the graphically emphasized element is detected on the touch sensitive display, the electronic controlling assembly creates and displays an updated screen on the touch sensitive display. On the updated screen, the emphasized element is changed to a graphical confirmation element representing confirmation of the touching and further changing another of the at least two graphical elements to be graphically emphasized. The updating of the screen with further detected touches of graphically emphasized elements is repeated until all graphical elements have been changed to represent graphical confirmation elements and finally followed by the full unlocking of the user interface.

The method may be amended in an alternative. In the alternative activation of the touch sensitive screen and before receiving the signal of the swipe movement, the electronic controlling assembly may create and display a status screen. The status screen may display information about past medicament or drug administration, information about the filling level of a medicament or drug reservoir present in the portable medical infusion pump. It further may comprise information about time and date and/or error message, battery status or any other information considered helpful (like received blood glucose measurement levels). It should be noted that the status screen may not only be shown in the event of the unlocking process, it may also make sense to show the status screen at other occasions. And the status screen may also be called in the menu of the portable medical infusion pump after the unlocking process is completed.

The method may be amended in an alternative. The described swipe movement may consist of a movement of a single finger of the user continuously touching the touch sensitive display from approximately a left edge of the touch sensitive display to approximately a right edge of the touch sensitive display. Alternatively, the swipe movement may be adapted to be from the right edge to the left edge, or from bottom edge to top edge, or from top edge to bottom edge. The swipe movement may be along a diagonal of the display. In even a further alternative, the touch sensitive display may be a so-called multi-touch display, capable of detecting touches of multiple fingers at the same time, which gives more alternatives to the swiping movement without departing from the invention.

The method may be amended in an alternative such that the medical infusion pump further comprises an acoustic emitter, such as buzzer or a speaker, controlled by the electronic controlling assembly. The electronic controlling assembly may be configured to create and emit one or more acoustic signals through the acoustic emitter during and/or after unlocking the user interface.

In a further amendment of the method the predefined amount of time the button may have to be pressed continuously to start the unlock process is at least one second or more. In alternatives the button is to be pressed between one and three seconds, or two and up to six seconds.

The method may be further and possibly amended such that the electronic controlling assembly is stopping the time needed for completing the unlock procedure. For example, if the described unlock procedure is not completed within a predefined duration limit, the electronic controlling assembly quits the unlock procedure and sets back the user interface into the locked state. The duration limit may be settable by the using person or a health care professional. A duration limit may be set during manufacturing of the portable medical infusion pump. This duration limit may be 30 second or shorter. In one alternative it may be 15 seconds or shorter. Optionally it may set to a value of 10 seconds.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts the portable medical infusion pump 1 in a perspective view with the touch sensitive display 10 switched off.
- Fig. 2: depicts the portable medical infusion pump 1 with the touch sensitive display 10 switched on but with user interface in a locked mode (ref. lock symbol 11d) showing the status screen 11.
- Figs 3a-3e: depicts the portable medical infusion pump 1 at different states of the unlock process in an embodiment of the invention.
- Fig. 4: depicts the portable medical infusion pump 1' during an alternative embodiment of the invention.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

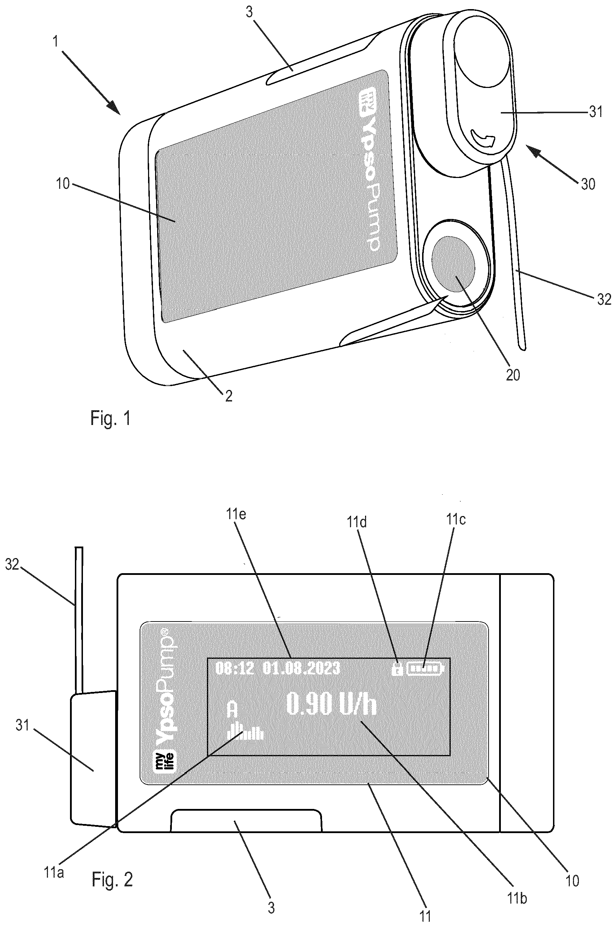

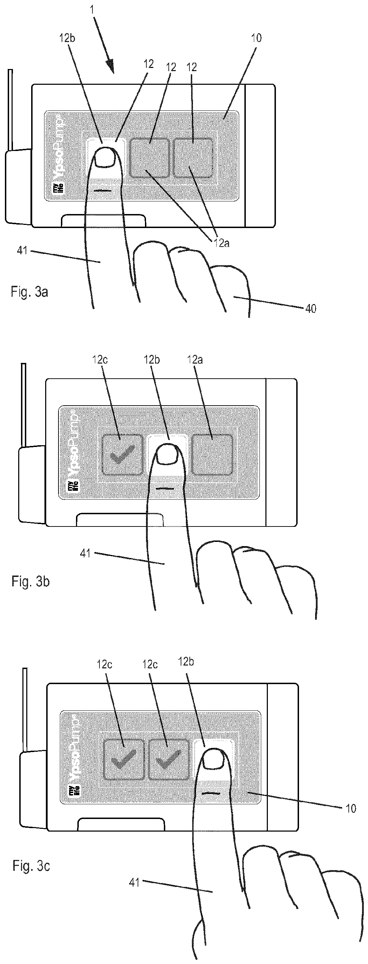

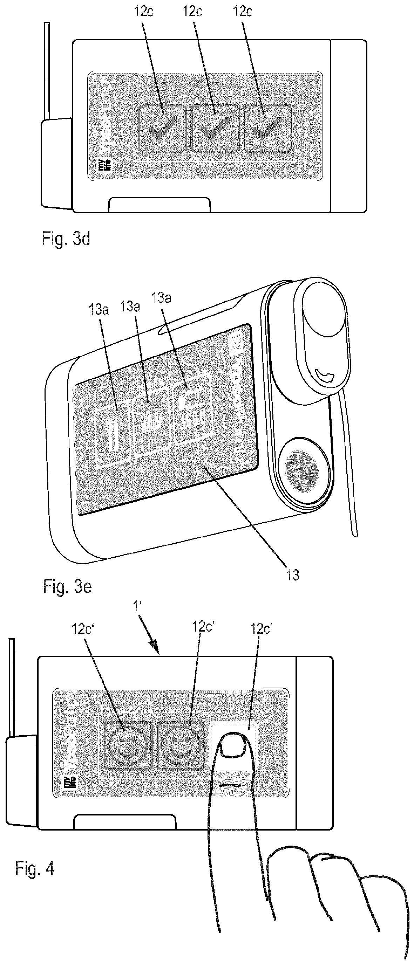

Figure 1 shows a perspective view of the portable medical infusion pump 1. Pump 1 is in this example an insulin pump of the type YpsoPump manufactured by Ypsomed. However, other types of pumps do not deviate from the invention. The portable medical infusion pump 1 according to the present invention comprises a user interface which comprises in the shown example of button 20 and the touch sensitive display 10. More components for the user interface are of course possible. The pump 1 further comprises a housing 2 with a window 3 which allows for a simple check of an inserted medicament cartridge (not shown), e. g. an insulin cartridge. Attached to the pump 1 is the infusion set 30, of which the infusion set adapter 31 and infusion tubing 32 are shown. The infusion set adapter 31 is coupled to the housing 2 of pump 1 and establishes a fluid path between the inserted medicament cartridge (not shown) and the infusion tubing 32. In figure 1, the pump 1 is shown in a state where the touch sensitive display is switched off and the user interface is in its locked state. As already described above the user interface may be put in a locked state and in an unlocked by the controlling assembly (not shown). In order to change the state from the locked into the unlocked state a using person has to interact with the user interface and to perform an unlock process. The unlock process is started by the using person by depressing button 20 with a finger 41 of hand 40 of the using person (hand 40 and finger 41 are shown e. g. on figure 3a), preferably for at least predefined duration. When the control assembly receives the according signal from button 20, it activates the touch sensitive display 10. Preferably (but not necessarily) the touch sensitive display 10 displays a status screen 11 as shown in figure 2. The status screen 11 comprises several elements to inform the using person about the current status of the portable medical infusion pump 1. The number and kind of elements shown may vary without deviation from the inventive idea. The status screen 11 in this example shows the currently running basal medicament profile 11a (e. g. basal insulin profile), the current infusion rate, 11b shown as insulin unit per hour (U/h), the battery status 11c, a lock symbol 11d (in case of locked user interface), and time and date 11e. In this state, the using person may proceed with the unlock process by swiping over the touch sensitive display 10 with a finger 41 from e. g. the left to the right end of the touch sensitive display 10. When receiving the swiping movement signal, the control assembly of the portable medical infusion pump 1 creates and displays the screen as shown in figure 3a. The touch sensitive display 10 displays several graphical elements 12. In this example three graphical elements 12. One of the graphical elements 12 is a graphically emphasized element 12b and the other two are not-emphasized graphical elements 12a. The graphical emphasis in figure 3a is added by filling the square of the graphical element and increasing the brightness of the graphical element. By this, attention is drawn to the graphically emphasized element 12b motivating the using person to touch the graphically emphasized element 12b with his or her finger 41. As the controlling assembly receives a signal from the touch sensitive display 10 which signals the touch of the graphically emphasized element 12b, it creates and displays another screen which is shown in figure 3b. This screen still shows three graphical elements 12, however, the most left one which used to be shown as graphically emphasized element 12b is changed into a graphical confirmation element 12c comprising a tick in the shown square signaling even illiterate users the completion of the process step. In addition, the graphical element in the middle of the screen on figure 3b is changed as well. Instead of a graphical element 12a (Figure 3a) an emphasized graphical element 12b is shown, again, attracting the attention of the using person. The third graphical element is still shown as graphical element 12a. If then the using person touches the newly emphasized element 12b, the control assembly receives the according signal and displays a further screen as shown in figure 3c. Analogously, as with the change from figure 3a to figure 3b, the graphically emphasized element 12b is changed into a graphical confirmation element 12c. Moreover, the next element (right side of the touch sensitive display 10) is changed into graphically emphasized element 12b. After the third graphically emphasized element 12b (fig. 3c) is touched by the using person, the unlock process is completed. The control assembly may then unlock the user interface and display the menu 13 of the portable medical infusion pump 1 as shown in figure 3e or optionally display the screen as shown in figure 3d where all graphical elements 12 are changed into graphical confirmation elements 12c symbolizing completion of the unlock process. The screen of figure 3d may be displayed for a pre-defined amount of time, e. g. one or two second, before the screen of figure 3e is shown which displays the pump menu 13 comprising the menu items 13a. If, during the unlock process, the using person touches for example a non-emphasized graphical element 12a or a graphical confirmation element 12c, the control assembly may detect an according signal but does not create and/or display a changed screen as it waits for a correct touch activity. Moreover, if a started unlock process is not completed by the using person within a pre-defined time window, the control assembly may switch off the touch sensitive display again and hence, cancel the unlock process. Furthermore, it may be possible to cancel the unlock process by the user. This may be possible by the user depressing button 20 after the touch sensitive display 10 was activated by the control assembly.

Figure 4 shows an alternative. The difference between the description of the figures 3a to 3d and the alternative of figure 4 is the different graphical confirmation element 12c'. The portable medical infusion pump 1' is very similar to the portable medical infusion pump 1. The difference may be limited to changes in the firmware installed on the control assembly. As can be seen in figure 4, the graphical confirmation element 12c' comprises a smiley-symbol instead of the tick in graphical confirmation element 12c. The unlock process itself may be identical to the one described for figures 3a to 3e.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

- 1: Portable medical infusion pump
- 1': Portable medical of alternative embodiment
- 2: Housing
- 3: Window
- 10: Touch sensitive display
- 11: Status screen
- 11a: Basal program
- 11b: Infusion rate
- 11c: Battery level
- 11d: Lock symbol
- 11e: Time and date
- 12: Graphical elements (group)
- 12a: (Single) graphical element
- 12b: Graphically emphasized element
- 12b': Graphically emphasized element
- 12c: Graphical confirmation element
- 12c': Alternative graphical confirmation element
- 13: Menu
- 13a: Menu item(s)
- 20: Button
- 30: Infusion set
- 31: Infusion set adaptor
- 32: Infusion tubing
- 40: Hand (of using person)
- 41: Finger

## Claims

1. A portable medical infusion pump suitable to infuse a fluid drug, the medical infusion pump comprising:
a. a user interface comprising a touch sensitive display allowing for user interaction with the medical infusion pump, and further comprising a button separate from the touch sensitive display,
b. an electronic controlling assembly, comprising a microcontroller or a processor, for operating and controlling the medical infusion pump,
c. the electronic controlling assembly is configured to put the user interface into a locked state and into an unlocked state,
wherein in the unlocked state a user may interact with the infusion pump through the user interface while in the locked stated the user may interact with the infusion pump through the user interface restrictedly only,
wherein the button is adapted to start an unlock process for the user interface, the unlock process comprising at least the following steps:
- pressing the button for at least a predefined amount of time by a user,
- activation of the touch sensitive display of the user interface by the electronic controlling assembly due to the signal received by the electronic controlling assembly from the button,
- swiping with a finger of the user over the touch sensitive display,
- creating and displaying a first screen on the touch sensitive display by the electronic controlling assembly after receiving the signal produced by the swiping, the first screen displaying at least two graphical elements, wherein one of the elements is graphically emphasized relative to the other elements,
- touching the graphically emphasized element with a finger of the user,
- after receiving the touching signal from the screen area of the graphically emphasized element by the electronic controlling assembly, the electronic controlling assembly creating and displaying an updated screen, with the graphically emphasized element changed to a graphical confirmation element representing confirmation of the touching and further changing another of the at least two graphical elements to be graphically emphasized,
- repeating the steps of touching and updating the screens until all of the graphical elements have been changed to represent graphical confirmation elements,
- finalizing the unlock process by unlocking the user interface.

2. The portable medical infusion pump of claim 1, wherein the graphical elements do not contain alphanumerical characters.

3. The portable medical infusion pump according to any of the preceding claims, wherein the graphical emphasis for the graphical emphasized element is realized by increasing the luminosity or changing the size, shape or color of the graphical element to be emphasized.

4. The portable medical infusion pump according to claims 1 or 2, wherein the graphical emphasis for the graphical emphasized element is realized by animating the graphical element to be emphasized.

5. The portable medical infusion pump according to any of the preceding claims, wherein the graphical elements prior to representing graphical confirmation elements consist of a simple geometrical and filled shape, such as a square, a rectangle, a triangle, optionally surrounded by a frame.

6. The portable medical infusion pump according to any of the preceding claims, wherein graphical confirmation elements comprise a check mark, a smiley, or a thumbs-up-symbol.

7. The portable medical infusion pump according to any of the preceding claims, wherein in the locked state the user may interact with the portable medical infusion pump for programming a blind bolus of the fluid drug, the blind bolus being programmed by repeatedly pressing the button in predefined sequences.

8. The portable medical infusion pump according to the preceding claim, wherein the number of button pressing events in the predefined sequences scales, preferably linearly, with the size of the bolus to be programmed.

9. A method to unlock the user interface of a portable medical infusion pump from a locked state, where only restricted interaction with a user is possible to an unlocked state where a user may fully operate and control the portable medical infusion pump, the medical infusion pump comprising:
- a user interface comprising a touch sensitive display and a button separate from the touch sensitive screen allowing for user interaction with the medical infusion pump,
- an electronic controlling assembly, at least comprising a microcontroller or a processor, for operating and controlling the medical infusion pump, the electronic controlling assembly is configured to receive signals from the button and the touch sensitive display and to create and display screens on the touch sensitive display,
wherein the electronic controlling assembly is further configured to perform at least the following steps in interaction with a user to unlock the user interface:
- starting the unlock procedure after the button is pressed for at least a predefined amount of time,
- activating of the touch sensitive display of the user interface,
- creating and displaying a first screen on the touch sensitive display after a swipe movement of a finger of the user is detected on the touch sensitive display, the first screen displaying at least two graphical elements, wherein one of the graphical elements is graphically emphasized relative to the other graphical elements,
- creating and displaying an updated screen on the touch sensitive display, after a touch of the graphically emphasized element is detected on the touch sensitive display, wherein on the updated screen the emphasized element is changed to a graphical confirmation element representing confirmation of the touching and further changing another of the at least two graphical elements to be graphically emphasized,
- repeating the updating to the screen with further detected touches of graphically emphasized elements until all graphical elements have been changed to graphical confirmation elements,
- unlocking of the user interface.

10. The method of claim 9, wherein the wherein in the electronic controlling assembly is further configured to create and display a status screen on the touch sensitive display after activating the touch screen but prior to detecting the swipe movement and prior to creating the displaying the first screen.

11. The method of claims 9 or 10, wherein the swipe movement consists of a movement of a single finger of the user continuously touching the touch sensitive display from approximately a left edge of the touch sensitive display to approximately a right edge of the touch sensitive display.

12. The method of any of the claims 9 to 11, wherein the medical infusion pump further comprises an acoustic emitter, such as buzzer or a speaker, controlled by the electronic controlling assembly, and wherein the electronic controlling assembly is configured to created and emit an acoustic signal through the acoustic emitter during or after unlocking the user interface.

13. The method according to any of the claims 9 to 12, wherein the button is pressed for at least a predefined amount of time and the predefined amount of time is one second or more.

14. The method according to any of the claims 9 to 12, wherein the button is pressed for at least a predefined amount of time and the predefined amount of time is two seconds or more.

15. The method according to any of the claims 9 to 14, wherein if the unlock procedure is not completed within 30 seconds, preferably 15 seconds, more preferably 10 seconds, the electronic controlling assembly is configured to cancel the unlock procedure and to re-lock the portable medical infusion pump in the locked state.
